# EUROPEAN PATENT SPECIFICATION

(11) **EP 2 247 320 B1**
(45) Date of publication and mention of the grant of the patent: **29.05.2013**
(21) Application number: 08728463.4
(22) Date of filing: 29.01.2008
(51) Int. Cl.: A61L 27/54, A61L 27/56, A61F 2/30

(54) **IMPLANT DEVICE FOR USE IN AN IMPLANT SYSTEM**
IMPLANTATVORRICHTUNG ZUR VERWENDUNG IN EINEM IMPLANTATSYSTEM
DISPOSITIF D'IMPLANT DESTINÉ À ÊTRE UTILISÉ DANS UN SYSTÈME D'IMPLANT

(43) Date of publication of application: 10.11.2010
(73) Proprietor: ZIMMER INC., Warsaw, Indiana 46580-2746 (US)
(72) Inventor: KLABUNDE, Ralf, CH-8405 Winterthur (CH); LÜSCHER, Patrick, CH-8330 Pfäffikon (CH)
(74) Representative: Mays, Julie
(86) International application number: PCT/US2008/052311
(87) International publication number: WO 2009/096945

(56) References cited:
- WO-A-2005/000159
- GB-A- 2 198 356
- US-A- 5 571 185
- US-B1- 6 409 764

## Description

### TECHNICAL FIELD

An implant device that releases a bioactive agent.

### BACKGROUND

Implant devices that release bioactive agents so that tissue reactions can be modified are of interest. One focus has been applying coatings onto implant surfaces that release bioactive agents. In the orthopedic field, a number of bioactive agents with the potential to accelerate or improve osseointegration of bone implants and bone repair are being investigated. Such agents include, e.g., bisphosphonates, parathyroid hormone, statins, and growth factors. Bone implant surfaces should be protected against microbial colonization, particularly for revision prostheses and trauma implants where infection rates may be up to 30%. While antibiotic-containing cements appear to have become the standard in Europe, there appears to be no accepted antibiotic technologies for non-cemented bone implants.

Bioactive agents usually require a delivery system to maintain a therapeutic concentration in the tissue because the agents are rapidly eliminated at the application site. Calcium phosphate coatings have been used as bioactive agent carrier systems. In such systems, bioactive agents are incorporated by adsorption to the implant surface. However, in this approach, loading and release characteristics are essentially defined by inherent surface interactions between the bioactive agent and the calcium phosphate substrate. Options to properly adjust the kinetics to the clinical requirements are limited. While intraoperative adsorption to calcium phosphate coatings has been suggested as an alternative to prefabricated combination products, this concept includes additional drawbacks, such as complexity of the operation and poor controllability.

The surfaces of spine and arthroplasty bone implants, for example, should be securely attached to surrounding bone tissue in order to transfer loads to the skeletal system. Currently, there are a number of surfaces used on bone implants that enable bone on- and in-growth that are either rough or porous and have long-term clinical records. Applying a coating onto such surfaces, however, incorporates the risk of impairing bone implant fixation and, thus, compromising clinical performance.

An implant device for covering bone implants that releases bioactive agents and allows for direct bone tissue integration with that bone implant, and that overcomes the above drawbacks and disadvantages of present implant devices and systems, is desirable.

### SUMMARY

An implant device for use in implant systems.

In one embodiment, the implant device includes a hollow body defining a covering with openings or reticulations, referred to as a mesh covering, including first and second opposing ends and an intermediate portion extending therebetween. At least one end defines an opening configured to receive a bone implant. In another embodiment, the implant device includes a planar body defining a mesh covering including upper and lower surfaces and an outer edge.

One or more bioactive agents are incorporated into or onto the hollow or planar body of the implant device. In one embodiment, the body includes a textile fabric such as a woven, knitted, and/or non-woven fabric, and/or a polymeric film. Each of the fabric and/or film has a plurality of openings, also referred to as reticulations. The openings, in one embodiment, include a maximum dimension of no greater than 20 mm and a minimum dimension no less than 0.2 mm.

The implant device is used in combination with a bone implant, e.g., a replacement hip joint stem or hip joint cup, to define an implant system. In one embodiment, the bone implant includes an outer surface that is generally covered by the implant device so that the hollow or planar body covers and conforms to at least a portion of the outer surface of the bone implant. The covering may cover all or part of the implant. In one embodiment, 99% to 20% of the covered outer surface of the bone implant is exposed through the openings in the mesh covering for direct bone tissue integration between adjacent bone tissue and the bone implant after implantation of the implant system in, or on, bone.

After implantation of the implant system, a desirable area of the outer surface of the bone implant is in direct contact with bone tissue. This secures primary stability and subsequent bone on- and in-gowth for secondary stability.

Various features discussed below in relation to one or more embodiments may be incorporated into any of the above-described embodiments alone or in any combination. The device and method will be further appreciated in relation to the following figures and description.

### BRIEF DESCRIPTION OF THE FIGURES

In the hollowing figures, like characters represent like parts throughout the figures.

FIG. 1 is a perspective view of an embodiment of an implant device;

FIG. 1A is an enlarged view of the implant device of FIG. 1 showing a body having a woven fabric;

FIG. 1B is an enlarged view of another embodiment of an implant device showing a knit fabric;

FIG. 1C is an enlarged view of another embodiment of an implant device showing a non-woven fabric with openings;

FIG. 1 D is an enlarged view of another embodiment of an implant device showing a polymeric film with openings;

FIG. 2 is a perspective view of the implant device of FIG. 1 covering a portion of a bone implant to define an implant system;

FIG. 3A is a side elevational view of the implant system of FIG. 2 implanted within a bone;

FIG. 3B is an enlarged cross-sectional view of the circled portion of FIG. 3A showing direct contact of adjacent bone tissue with the exposed surface of the bone implant through openings in the implant device for bone tissue integration;

FIG. 4 is a perspective view of another embodiment of an implant device;

FIG. 5A is a perspective view of the implant device of FIG. 4 ready for insertion into a prepared cavity in a bone;

FIG. 5B is a perspective view of the implant device of FIG. 4 being inserted into the prepared cavity;

FIG. 5C is a perspective view of the implant device of FIG. 4 implanted in the bone and the bone implant received within the implant device to define an implant system;

FIG. 6 is a perspective view of a circular embodiment of the implant device of FIG. 4 being sandwiched between a tibia and bone implant to define an implant system;

FIG. 7A is a perspective view of a circular embodiment of the implant device of FIG. 4 situated adjacent a bone implant; and

FIG. 7B is a perspective view of the implant device of FIG. 7A received over the bone implant to define an implant system.

### DETAILED DESCRIPTION

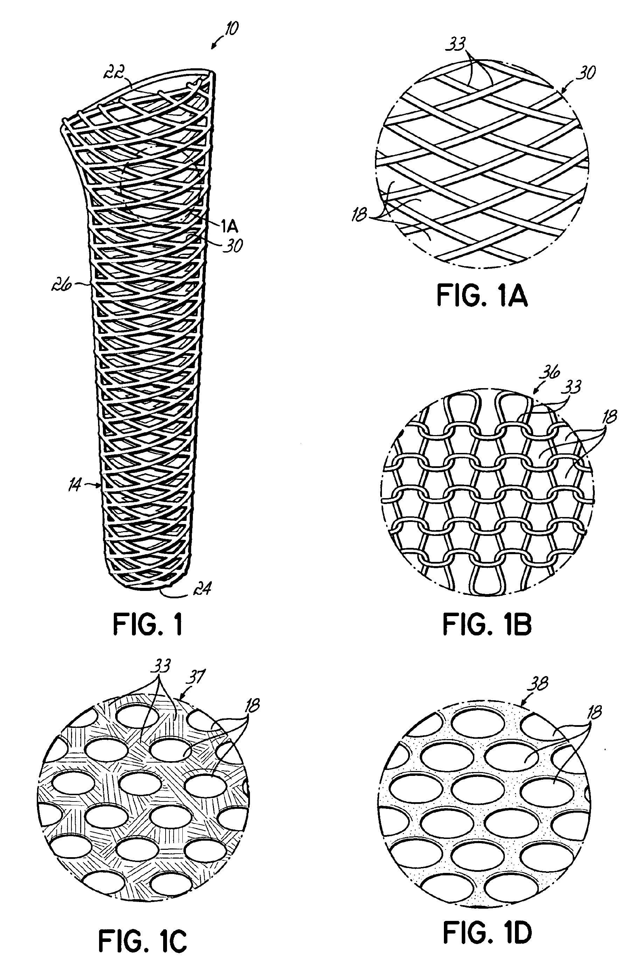

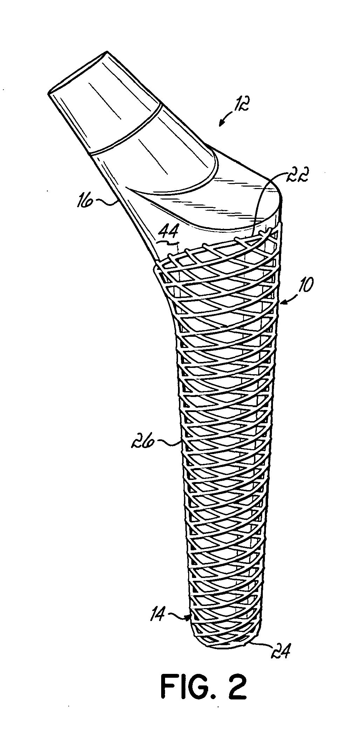

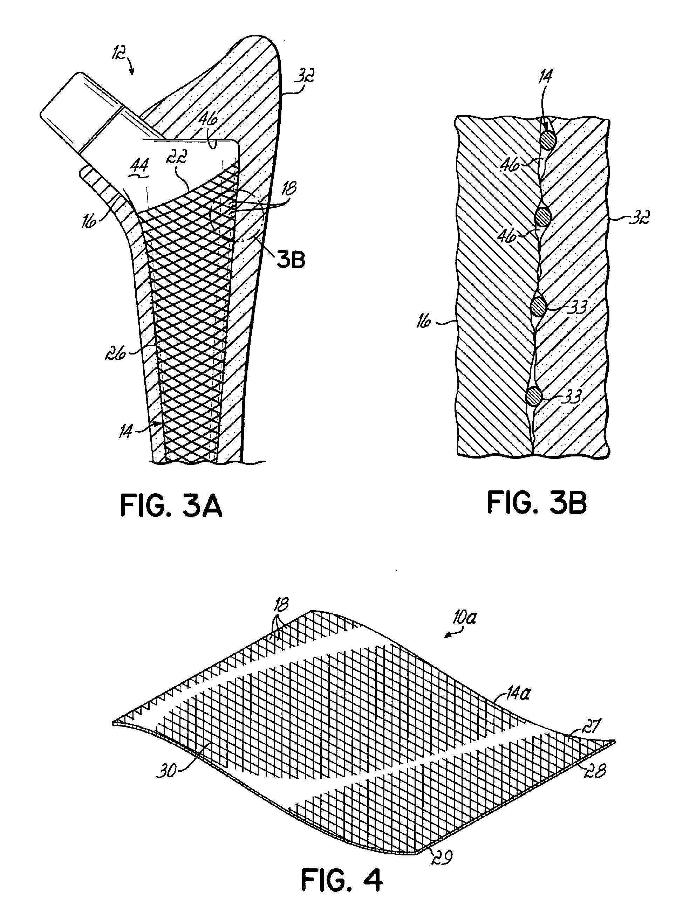

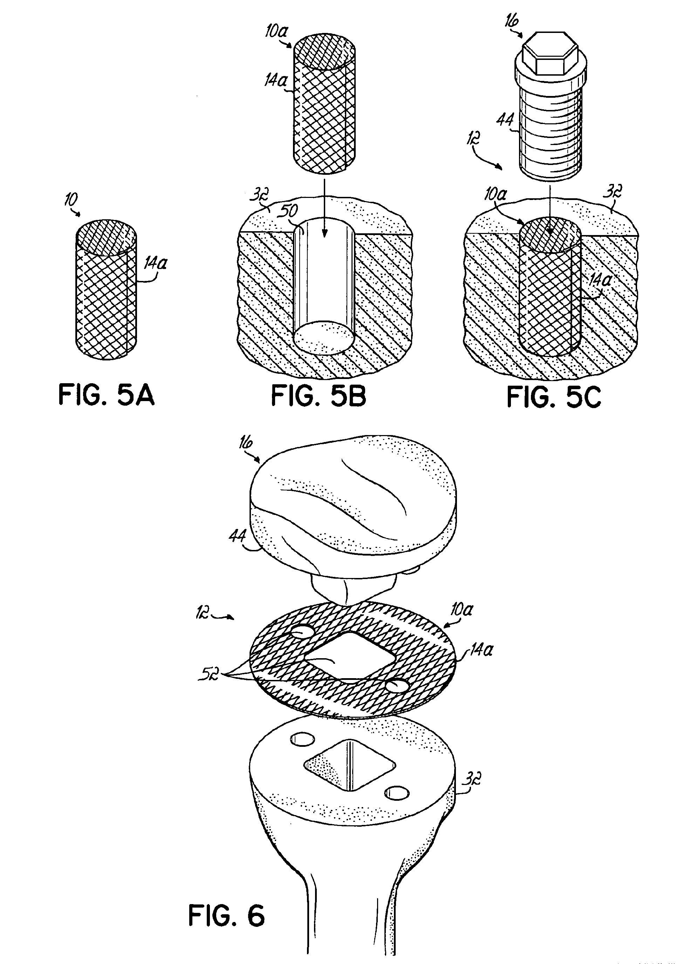

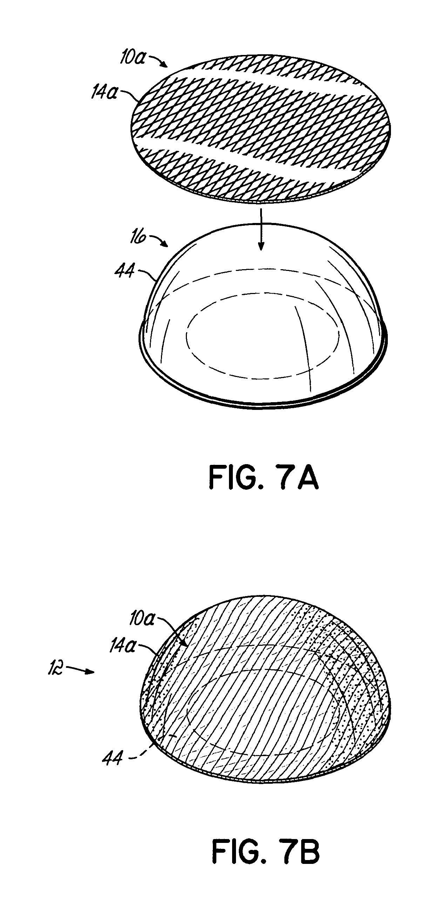

FIGS. 1-7B show embodiments that relate to an implant device 10, 10a for use in an implant system 12. Such implant device 10, 10a includes and can release a Bioactive agent(s) and also includes a body 14, 14a defining a mesh covering for covering a bone implant 16, such as a replacement hip joint stem (See FIG. 2), a bone screw (See FIG. 5C), a knee replacement component (See FIG. 6), or a hip joint cup (See FIGS. 7A and 7B). The implant device 10, 10a and bone implant 16 together define implant system 12 where a plurality of desirably sized openings 18 in the covering allow for direct bone tissue integration with the bone implant 16 after implantation of the implant system 12. The body 14, 14a of the implant device 10, 10a may be hollow-like (See, e.g., FIG. 1) or planar (See, e.g., FIG. 4).

With specific reference to FIGS. 1 and 1A, in one embodiment, the implant device 10 includes hollow body 14 defining an elongated mesh covering including first and second opposing ends 22 and 24 and an intermediate portion 26 extending therebetween. The first end 22 defines an opening configured to receive the bone implant 16 and the second end 24 defines a closed end. One of ordinary skill in the art will appreciate that the second end 24 may define an opening rather than a closed end.

In another embodiment and with specific reference to FIG. 4, implant device 10a includes planar body 14a defining a mesh covering including upper and lower surfaces 27 and 28 and an outer edge 29. Although shown generally in the shape of a rectangle, it should be understood that the implant device 10a may generally take on any number of shapes, such as circular (See FIGS. 6, 7A, and 7B), for example.

While implant device 10 is discussed in detail next, the details set out apply equally to implant device 10a, particularly with respect to discussion of hollow body 14 which may be interchanged with planar body 14a, unless otherwise noted.

With reference again to FIGS. 1 and 1A, the hollow body 14 includes a woven fabric 30 having the plurality of openings 18 therein to form the mesh covering and to allow, as shown in FIGS. 3A and 3B and further described below, for direct contact between bone implant 16 and bone 32 for bone tissue integration after implantation of the implant system 12. The weave of the woven fabric 30 may include a braid to define a braided fabric with openings 18. The openings 18 in the hollow body 14 are defined by the spacing between yarns 33. Such yarns 33 may generally include fibers, filaments (mono- or multifilament), or other material understood as being suitable for use in woven fabrics in the field of bone implants.

In an alternate embodiment, as shown in FIG. 1B, the hollow body 14 includes a knitted fabric 36 having the plurality of openings 18. The openings 18 in the hollow body 14, likewise, are defined by the spacing between yarns 33 in the knitted fabric 30. In another embodiment, as shown in FIG. 1C, the hollow body 14 includes a non-woven fabric 37, such as a staple non-woven or spun-laid non-woven, e.g., electrospun non-woven, provided with a plurality of openings 18 therethrough. In yet another embodiment, as shown in FIG. 1D, the hollow body 14 includes a polymeric film 38 provided with a plurality of openings 18 therethrough.

In one embodiment, the openings 18 in the hollow body 14 include a maximum dimension of no greater than 20 mm and a minimum dimension no less than 0.2 mm. In another embodiment, openings 18 may include a maximum dimension of no greater than 5 mm and a minimum dimension no less than 0.5 mm. In yet another embodiment, openings 18 may include a maximum dimension of no greater than 5 mm and a minimum dimension no less than 1 mm. In still another embodiment, openings 18 may include a dimension of 0.5 mm. The openings 18 may be symmetrical and/or asymmetrical and may be evenly or unevenly spaced apart. In one embodiment, the dimensions of the openings maybe variable. Such openings 18, as further explained below, allow for a desirable amount of exposure of the bone implant 16 for direct bone tissue integration to provide secure attachment of the bone implant 16 to the bone 32.

At least one bioactive agent is incorporated into or onto the woven, knit, and/or non-woven fabric 30, 36, 37 or polymer film 38, as described further below.

Fibers of the woven, knit, and non-woven fabric 30, 36, 37 which form the yarns 33 therein, as well as the polymer film 38 may include non-biodegradable polymers (e.g., polymethyl methacrylate, polyethylene, polyurethane, etc.), biodegradable polymers (e.g., pentacosadiynoic acid, poly(lactic acid), poly(glycolic acid), poly(lactic acid-glycolic acid), poly(glaxanone), poly(orthoesters), poly(pyrolic acid), and poly(phosphazenes)), and/or biopolymers (e.g., collagen fibers), and the like. The fibers also may include metal (e.g., magnesium alloys), ceramics (e.g., calcium phosphate fibers), and glass (e.g., bioglass fibers as disclosed in WO 2004/031086. The polymer(s) may be selected, for example, based on the desired degradation characteristics and the desired bioactive agent to be incorporated in, or on, the hollow body 14. To make biocompatible polymers, residual solvent may need to be removed, using methods known in the art.

The woven and knit fabric 30, 36 for the hollow body 14 may be prepared using standard weaving, braiding, and/or knitting techniques known to one of ordinary skill in the art. For example, the fabric 30, 36 may be constructed by intertwining or orthogonal interlacing of yarns 33 to form an integral structure through position displacement. A wide range of three-dimensional fabrics may be fabricated in a circular or rectangular loom. The dimensions of the openings 18 may be varied as needed using, for example, standard weaving, braiding, and/or knitting techniques to obtain a desired percent exposure of the bone implant 16 when the implant device 10 covers the bone implant 16. The percent exposure of the bone implant 16 is further described below.

The nonwoven fabric 37 for the hollow body 14 may be prepared using standard nonwoven techniques known to one of ordinary skill in the art. For example, the non-woven fabric 37, which generally is an assembly of textile fibers held together by mechanical interlocking in a random web or mat, may be prepared by fusing of the fibers, or by bonding with a cementing medium such as starch, glue, casein, rubber, latex, or one of the cellulose derivatives or synthetic resins. The openings 18 in the nonwoven fabric 37 may be formed by known techniques, such as by cutting or punching holes in the resulting fabric 37, to obtain a desired percent exposure of the bone implant 16 when the implant device 10 covers the b one implant 16.

Hollow bodies 14 that include the polymeric film 38 may be prepared by methods known to one of ordinary skill in the art. In one embodiment, the polymeric film 38 may be made by heat pressing and melt forming/drawing methods known in the art. In one embodiment, the polymeric film 38 maybe formed into a conical shape to be drawn over a portion of bone implant. Thicker films can be pressed to form thinner films, and can be drawn out after heating and pulled over forms of the desired shapes, or pulled against a mold by vacuum pressure. In addition, the openings 18 in the polymeric film 38 may be formed, and manipulated as desired, by known foaming techniques or incorporation of porogenic materials, such as leachable salts or laser etching. Other methods known to one of ordinary skill in the art may be used to make openings of the desired size in the hollow body 14. For example, the polymeric film 38 may be formed by molding techniques, which provides the plurality of openings 18 in the film 38. While the openings 18 may be formed in the film 38 prior to forming the hollow body 14 into the shape as shown, it is understood that the openings 18 may be formed afterwards.

Hollow body 14 may be produced so that different areas of the body 14 have different properties, such as different degradation rates, thicknesses, bioactive agents, and/or opening sizes, which could affect bone in-growth, cell attachment, drug-release kinetics, etc. To make woven, knit, and non-woven fabrics having different characteristics in different areas, different fibers, fiber thicknesses, non-woven thicknesses, fabric structures and/or different bioactive agent concentrations or compositions may be used In particular, to make polymeric films 38 having different characteristics in different areas, separate films, each having desired properties, can be made and cut to shape. The shapes can then be integrated, such as heat-welding together, by overlapping the sections, for example, by at least 2 mm then applying pressure, such as gentle pressure, at a suitable temperature, such as 60°C.

The hollow body 14 also may have an elastic nature so that it adjusts to non-linear, e.g., curved, bone implants 16 without draping and so that is more readily conforms to different bone implant sizes and/or designs. The hollow body 14 also can be used over smooth, rough, or porous surfaces of the bone implant 16 and may be cut intraoperatively to fit the size and/or shape of the bone implant 16.

Bioactive agents can be incorporated into or onto the hollow body 14 such as by directly incorporating the bioactive agents into the material that forms the hollow body 14 or by attachment of the bioactive agent onto the surface of the hollow body 14. In one embodiment, bioactive agents may be added directly to polymer material before the fibers for the fabric 30, 36, 37 or the film 38 is made, thereby creating fabric 30, 36, 37 or film 38 that directly incorporates the bioactive agent. In another embodiment, fabric 30, 36, 37 or film 38 may be placed in solutions containing bioactive agents allowing the bioactive agent to be adsorbed onto the surface thereof. In another embodiment the bioactive agent may be chemically bonded to the surface of fabric 30, 36, 37 or film 38. The bioactive agent can be released by dissolution, desorption, diffusion, or degradation of the carrier material or the chemical bond. The fabric 30, 36, 37 may include only bioactive agent-containing fibers, bioactive agent-containing fibers sandwiched between fibers that contain no bioactive agent, mixtures of fibers containing different bioactive agents, fibers that contain no bioactive agent, or combinations.

The bioactive agents incorporated into or onto the body 14 can include, e.g., a drug or biological factor, such as an osteogenic agent and/or an osteoinductive agent, that can promote bone growth and/or healing, thus enhancing the overall healing characteristics of the bone implant. Such osteogenic and osteoinductive agents can include, e.g., members of the families of bone morphogenetic proteins (BMPs), osteoprotegerin or any of the other osteoclastogenesis inhibitors, proteasome inhibitors, connective tissue growth factors (CTGFs), vascular endothelial growth factors (VEGFs), transforming growth factors-β (TGF-βs), growth differentiation factors (GDFs), cartilage derived morphogenic proteins (CDMPs), and lim mineralization proteins (LMPs). Osteoconductive agents may optionally be provided with the body 14 with the osteogenic and/or osteoinductive agents.

BMPs are a class of proteins thought to have osteoinductive or growth-promoting activities on endogenous bone tissue, or function as pro-collagen precursors. Known members of the BMP family that may be used as osteoinductive agents in tissue attachment formulations include BMP-1, BMP-2, BMP-3, BMP-4, BMP-5, BMP-6, BMP-7, BMP-8, BMP-9, BMP-10, BMP-11, BMP-12, BMP-13, BMP-15, BMP-16, BMP-17, and BMP-18 polynucleotides and polypeptides, as well as mature polypeptides and polynucleotides encoding them. The BMPs may be included with the body 14 as full length BMPs or fragments thereof, or combinations or mixtures thereof, or as polypeptides or polynucleotides encoding the polypeptide fragments of all of the recited BMPs, as disclosed in Termaat et al., J Bone Joint Surg Am., 87 (2005)1367.

Osteoclastogenesis inhibitors inhibit bone resorption by osteoclasts of the bone tissue surrounding the implantation site. Osteoclast and osteoclastogenesis inhibitors include osteoprotegerin polynucleotides and polypeptides, as well as mature osteoprotegerin polypeptides, and polynucleotides encoding them. The osteoprotegerin protein specifically binds to its ligand, osteoprotegerin ligand (TNFSF11/OPGL), both of which are key extracellular regulators of osteoclast development. Osteoclastogenesis inhibitors further include chemical compounds such as bisphosphonates (e.g., alendronate, clodronate, etidronate, ibandronate, (3-amino-1-hydroxypropylidene)-1,1-bisphosphonate (APD), dichloromethylene bisphosphonate, aminobisphosphonatezolendronate, zoledronic acid, and pamidronate, disclosed in Morris et al., J Bone Joint Surf Am., 87 (2005) 1609, , 5-lipoxygenase inhibitors such as those described in U.S. Patent Nos. 5,537 ,524 and 6,455,541, heterocyclic compounds such as those described in U.S. Patent No. 5,658,935), 2,4-dioxoimidazolidine and imidazolidine derivative compounds such as those described in U.S. Patent Nos. 5,397,796 and 5,554,594, sulfonamide derivatives such as those described in U.S. Patent No. 6,313,119, and acylguanidine compounds such as those described in U.S. Patent No. 6,492,356,

CTGFs are a class of proteins having growth-promoting activities on connective tissues. Known members of the CTGF family include CTGF-1, GTGF-2, and CTGF-4, any of which may be incorporated into or onto the body 14, in addition to polypeptides and/or polynucleotides encoding them.

VEGFs are a class ofproteins having growth-promoting activities on vascular tissues. Known members of the VEGF family include VEGF-A, VEGF-B, VEGF-C, VEGF-D and VEGF-E, any of which may be incorporated into or onto the body 14 separately or in combination, in addition to polypeptides and polynucleotides encoding them.

TGF-βs are a class of proteins having growth-promoting activities on a range of tissues, including connective tissues. Known members of the TGF-β family include TGF-β-1, TGF-β-2, and TGF-β-3, any of which may be incorporated into or onto the body 14 separately or in combination, in addition to polypeptides and polynucleotides encoding them

Known GDFs include GDF-1, GDF-2, GDF-3, GDF-7, GDF-10, GDF-11, and GDF-15. GDF-1 polynucleotides and polypeptides generally correspond to GenBank Accession Nos. M62302, AAA58501, and AAB94786; GDF-2 polynucleotides and polypeptides correspond to GenBank Accession Nos. BC069643, BC074921, Q9UK05, AAH69643, and AAH74921; GDF-3 polynucleotides and polypeptides correspond to GenBank Accession Nos. AF263538, BC030959, AAF91389, AAQ89237 , and Q9NR23; GDF-7 polynucleotides and polypeptides correspond to GenBank Accession Nos. AB158468, AF522369, AAP97720, and Q7Z4P5; GDF-10 polynucleotides and polypeptides correspond to GenBank Accession Nos. BC028237 and AAH28237; GDF-11 polynucleotides and polypeptides correspond to GenBank Accession Nos. AF100907, NP005802 and 095390; and GDF-15 polynucleotides and polypeptides correspond to GenBank Accession Nos. BC008962, BC000529, AAH00529, and NP004855.

Known CDMPs and LMPs include CDMP-1, CDMP-2, LMP-1, LMP-2, and LMP-3. CDMP-1 polynucleotides and polypeptides generally correspond to GenBank Accession Nos. NM000557, U13660, NP000548 and P43026; CDMP-2 polypeptides correspond to GenBank Accession No. P55106; LMP-1 polynucleotides and polypeptides correspond to GenBank Accession Nos. AF37 5904 and AAK30567; LMP-2 polynucleotides and polypeptides correspond to GenBank Accession Nos. AF37 5905 and AAK30568; and LMP-3 polynucleotides and polypeptides correspond to GenBank Accession Nos. AF37 5906 and AAK30569.

Additional osteoinductive and osteoconductive agents, factors, and compounds that can also be used as bioactive agents include hydroxyapatite (HA), tricalcium phosphate (TCP), collagen, fibronectin (FN), osteonectin (ON), endothelial cell growth factor (ECGF), cementum attachment extracts (CAE), ketanserin, human growth hormone (HGH), animal growth hormones, parathyroid hormone (PTH) (Aleksyniene and Hvid, Medicina (Kaunas), 40, 842-849, 2004), epidermal growth factor (EGF), interleukin-1 (IL-1), human alpha thrombin, insulin-like growth factor (IGF-1), platelet derived growth factors (PDGF), fibroblast growth factors (FGF, βFGF, etc.), proteasome inhibitors (Garrett et al.: Selective inhibitors of the osteoblast proteasome stimulate bone formation in vivo and in vitro. J Clin Invest. 2003; 11:1771-1782) and molecules (e.g. Wnt-proteins), which are involved in the regulation of the osteoblastic lineage and its function. Among those pathways are the canonical Wnt/β-catenin pathway, sonic hedgehog and the BMP pathway via SMAD1/5.

Other examples of bioactive agents include glycogen synthase kinase 3 (GSK-3) inhibitors, biocidal/biostatic sugars such as dextran and glucose, vitamins, cartilage fragments, natural extracts, genetically engineered or otherwise modified living cells, permeation/penetration enhancers such as fatty acid esters including laureate, myristate, and stearate monoesters ofpolyethylene glycol, salts such as strontium salt, fluoride salt, magnesium salt, and sodium salt, bone marrow aspirate, and/or bone marrow concentrate.

Bioactive agents that are full-length proteins or fragments may be conjugated to polyethylene glycol (PEG) moieties, i.e., pegylation, to increase their half-life *in vivo.* Methods of pegylating polypeptides are known by one of ordinary skill in the art. In addition, bioactive agents may be delivered by gene therapy vectors harboring the polynucleotides encoding them. The vector maybe, e.g., a phage, plasmid, viral, or retroviral vector. Such gene therapy and delivery techniques are known in the art. Gene therapy vectors further comprise suitable adenoviral vectors. Suitable gene therapy vectors include gene therapy vectors that do not integrate into the host genome and gene therapy vectors that integrate into the host genome. A desired polynucleotide also maybe delivered in plasmid formulations. Plasmid DNA or RNA formulations refer to polynucleotide sequences encoding osteoinductive polypeptides that are free from any delivery vehicle that acts to assist, promote, or facilitate entry into the cell, including viral sequences, viral particles, liposome formulations, lipofectin or precipitating agents, etc.

The bioactive agents may be available as heterodimers or homodimers, and/or multimers. Recombinantly expressed proteins may be in native forms, truncated analogs, muteins, fusion proteins (e.g., fusion proteins with the FC portion of human IgG), and other constructs capable ofinducing bone, cartilage, or other types of tissue formation as demonstrated by in *vitro* and *ex vivo* bioassays and *in vivo* implantation in mammals, including humans. Examples of fusion proteins include ligand fusions between mature osteoinductive polypeptides and the FC portion of human Immunoglobulin G (IgG). Methods of making fusion proteins and constructs encoding them are known in the art.

Examples of suitable drugs include antitumor agents and chemotherapeutics such as cisplatin, ifosfamide, methotrexate, bortezomib and doxorubicin hydrochloride, inmuno-suppressants, statins, pain killers and anti-inflammatories such as non-steroidal antiinflammatory drugs (NSAID) such as ketorolac tromethamine, lidocaine hydrochloride, bipivacaine hydrochloride, and ibuprofen, antibiotics or other bactericidal agents, and antiretroviral drugs. Bactericidal drugs and antiretroviral dmgs may be provided to prevent infection by pathogens that are introduced to the patient during implant surgery. Administration of antibiotics and antiretroviral drugs also may be useful to account for nosocomial infections or other factors specific to the location where implant surgery is conducted. Antibiotics and antiretroviral drugs include aminoglycosides such as tobramycin, amoxicillin, ampicillin, azactam, bacitracin, beta-lactamases, beta-lactam (glycopeptide), biomycin, clindamycin, chloramphenicol, chloromycetin, cefazolin, cephalosporins, ciprofloxacin, erythromycin, fluoroquinolones, gentamicin, macrolides, metronidazole, neomycin, penicillins, polymycin B, quinolones, rapamycin, rifampin, streptomycin, sulfonamide, tetracyclines, trimethoprim, trimethoprim-sulfamethoxazole, vancomycin, and mixtures and combinations thereof. Bactericidal agents include the group of metal ions such as silver and copper.

The fabric 30, 36, 37 or polymeric film 38, can be combined with the bone implant 16 and implanted into a patient for release of bioactive agents, as the fabric 30, 36, 37 or film 38 degrades and/or as the bioactive agent diffuses from the fabric 30, 36, 37 or film 38, for example. The delivery rate of the bioactive agent can be controlled by varying the choice of material, e.g., polymer, used in the fabric 30, 36, 37 or polymeric film 38, the concentration of material used in the fabric 30, 36, 37 or polymeric film 38, the diameter of the fiber of the fabric 30, 36, 37 and/or the amount of bioactive agent loaded in, or on, the fabric 30, 36, 37 or polymeric film 38.
The amount or concentration of bioactive agent to be incorporated into or coated on the fabric 30, 36, 37 and/or polymeric film 38 of the hollow body 14 can be any amount that shows a measurable effect in improving the performance of the covered bone implant 16, as known by one of ordinary skill in the art or determined by testing the implant system 12 with and without the bioactive agent(s) and measuring at least one characteristic to be improved including, but not limited to, decrease of infection rate, higher bone apposition index, faster secondary fixation, or longer survival time.

With further reference to FIGS. 2, 3A, and 3B, the hollow body 14 has a shape sufficient to cover at least a portion of the bone implant 16. In particular, the bone implant 16 includes an outer surface 44 and is received within the opening of the first end 22 so that the hollow body 14 covers and conforms to at least the portion of the outer surface 44 of the bone implant 16. In one embodiment, 99% to 20% of the covered outer surface 44 of the bone implant 16 is exposed through the openings 18 in the mesh covering for direct bone tissue integration after implantation of the implant system 12. In another embodiment, 99% to 40% of the covered outer surface 44 of the bone implant 16 is exposed through the openings 18 in the mesh covering for direct bone tissue integration.

With specific reference to FIGS. 3A and 3B, the hollow body 14 of the implant device 10 may be situated about the bone implant 16 before the bone implant 16 is placed in a prepared cavity 46 of a bone 32, e.g., a femur. Alternatively, the hollow body 14 maybe inserted into the prepared cavity 46 in the bone 32 before the bone implant 16 is inserted. After implanting the implant system 12 in the bone 32, a desirable area of the outer surface 44 of the bone implant 16 is exposed and in direct contact with the bone 32. This facilitates direct implant-to-bone contact for secure primary stability and subsequent bone on- and in-growth for secondary stability. In this embodiment, there is no intervening layer(s) between the bone 32 and the surface 44 of the bone implant 16 that impedes the natural process of osseointegration.

With further reference now to FIGS. 4-7B, implant device 10a includes planar body 14a that has woven fabric 30 with openings 18 therein to form the mesh covering and which allows for direct contact between bone implant 16 and bone 32. In alternate embodiments, as indicated above, the planar body 14a can include knitted fabric 36, non-woven fabric 37, and/or polymeric film 38 having the plurality of openings 18 therethrough. Bioactive agents are incorporated into or onto the planar body 14a to promote bone tissue integration after implantation of the implant system 12. The planar body 14a has a size and shape sufficient to cover at least a portion of bone implant 16 that comes into contact with bone 32. For example, the planar body 14a may be wrapped around, placed over, or sandwiched between the outer surface 44 of the bone implant 16 and the bone 32, as discussed next. Accordingly, one skilled in the art will appreciate that the implant device 10a may be shaped or configured in any variety of ways for use with bone implants 16 which can be used in, or on, any variety of bones. Specific non-limiting examples are discussed next.

With reference to FIG. 5A, the planar body 14a of implant device 10a can be rolled into a tube to receive bone implant 16, i.e., a bone screw (See FIG. 5C). That rolled implant device 10a, as shown in FIG. 5B, is inserted into prepared cavity 50 of bone 32. Alternately, one skilled in the art will appreciate that the planar body 14a of implant device 10a may be placed about the bone implant 16 prior to insertion thereof into the prepared cavity 50. Once the implant device 10a is in place, the bone implant 16, as shown in FIG. 5C, is inserted, or screwed, into the cavity 50 thus defining implant system 12. Accordingly, the planar body 14a is positioned between the bone implant 16 and the bone 32 to allow for bone tissue integration.

As shown in FIG. 6, the planar body 14a of implant device 10a is circular-shaped and includes apertures 52 that conform to the shape of bone implant 16, i.e., a tibial knee component, to define implant system 12. The implant device 10a is sandwiched between the bone implant 16 and bone 32, i.e., a tibia, which allows for bone tissue integration therebetween. One skilled in the art will appreciate that the planar body 14a may be placed either on the bone implant 16 or on the bone 32 prior to placement of the bone implant 16. In addition, the planar body 14a may be precut or cut intraoperatively to the desired shape.

As shown in FIGS. 7A and 7B, the planar body 14a of implant device 10a is circular-shaped to conform to bone implant 16, i.e., a hip cup, to define implant system 12. In particular, the implant device 10a is placed over the bone implant 16 to cover the outer surface 44 of the bone implant 16 then secured within bone (not shown), e.g., a hip socket, as known in the art, which allows for bone tissue integration between the bone implant 16 and bone. In the alternative, the implant device 10a may be inserted into the hip socket prior to the placement of the bone implant 16.

After the implant system 12 is implanted in the bone 32, a desirable area of the outer surface 44 of the bone implant 16 is exposed and in direct contact with the bone 32. And, this facilitates direct implant-to-bone contact for secure primary stability and subsequent bone on- and in-growth for secondary stability. As discussed above, 99% to 20% of the covered outer surface 44 of the bone implant 16 is exposed through the openings 18 in the mesh covering for direct bone tissue integration after implantation of the implant system 12. In another embodiment, 99% to 40% of the covered outer surface 44 of the bone implant 16 is exposed through the openings 18.

While the bone implants 16 are generally depicted herein as a replacement hip joint stem, bone screw, tibial knee component, and hip cup, one skilled in the art will appreciate that the bone implant 16 generally may be one suited for placement in or on virtually any desired bone. Various sizes and shapes of the bone implant 16 are employed as needed. In addition, the bone implant 16 may be composed of hydroxylapatite, titanium, cobalt chrome, stainless steel, silicon, ceramics, polyvinyl chlorate, and/or other polymers, or may be composed of biodegradable materials such as poly(lactic acid):poly(glycolic acid) implants as described in U.S. Patent No. 5,716,413.

Various changes can be made in the above-described embodiments without departing from the scope of the invention. Thus, the above description and figures are illustrative and not limiting.

## Claims

1. An implant device comprising:
a hollow or planar body comprising (a) a textile fabric and/or (b) a polymeric film which defines a mesh covering, and one or more bioactive agents incorporated into or onto the hollow or planar body, wherein (a) and (b) each include a plurality of openings, the openings having a maximum dimension of no greater than 20 mm and a minimum dimension no less than 0.2 mm.

2. The implant of claim 1 where the openings have a maximum dimension no greater than 5 mm and a minimum dimension no less than 0.5 mm.

3. The implant of claim 1 wherein the hollow or planar body comprises (a) the woven, knitted or nonwoven fabric.

4. The implant of claim 1 wherein the hollow or planar body comprises (b) the polymeric film.

5. The implant of claim 1 in which (a) and/or (b) is biodegradable.

6. The implant device of claim 1 wherein the hollow body defines the mesh covering which includes first and second opposing ends and an intermediate portion extending therebetween, at least one end defining an opening configured to receive a bone implant.

7. The implant of claim 6 wherein the first end defines an opening configured to receive a bone implant and the second end defines a closed end.

8. The implant device of claim 1 wherein the planar body defines the mesh covering which includes upper and lower surfaces and an outer edge, the planar body configured to cover and conform to at least a portion of an outer surface of a bone implant.

9. An implant system comprising:
an implant device as defined in claim 1, and
a bone implant including an outer surface, the hollow or planar body covering and conforming to at least a portion of the outer surface of the bone implant wherein 99% to 20% of the covered outer surface of the bone implant is exposed through the openings in the mesh covering for desirable bone tissue integration.

10. The implant system of claim 9 wherein 99% to 40% of the covered outer surface of the bone implant is exposed through the openings in the mesh covering for bone tissue integration.

11. The implant system of claim 9 wherein the implant device includes the hollow body defining the mesh covering which includes first and second opposing ends and an intermediate portion extending therebetween, at least one end defining an opening configured to receive the bone implant, and wherein the bone implant is received within the opening so that the hollow body covers and conforms to at least a portion of the outer surface of the bone implant.

12. The implant system of claim 11 wherein the first end defines an opening configured to receive the bone implant and the second end defines a closed end.

13. The implant system of claim 9 wherein the implant device includes the planar body defining the mesh covering which includes upper and lower surfaces and an outer edge, the planar body covering and conforming to at least a portion of the outer surface of the bone implant.

## Patentansprüche

1. Implantatvorrichtung mit:
Einem Hohl- oder Flachkörper, der aufweist: (a) eine Textilware und/oder (b) eine Polymerfolie, die einen Netzgewebeüberzug definiert, und einen oder mehrere bioaktive Wirkstoffe, die in oder an dem Hohl- oder Flachkörper eingearbeitet sind, wobei (a) und (b) jeweils mehrere Öffnungen aufweisen, wobei die Öffnungen eine maximalen Abmessung von nicht mehr als 20 mm und eine minimale Abmessung von nicht weniger als 0,2 mm haben.

2. Implantatvorrichtung nach Anspruch 1, wobei die Öffnungen eine maximale Abmessung von nicht mehr als 5 mm und eine minimalen Abmessung von nicht weniger als 0,5 mm haben.

3. Implantatvorrichtung nach Anspruch 1, wobei der Hohl- oder Flachkörper (a) eine Web-, Wirk- oder Vliesware aufweist.

4. Implantatvorrichtung nach Anspruch 1, wobei der Hohl- oder Flachkörper (b) die Polymerfolie aufweist.

5. Implantatvorrichtung nach Anspruch 1, wobei (a) und/oder (b) biologisch abbaubar sind.

6. Implantatvorrichtung nach Anspruch 1, wobei der Hohlkörper den Netzgewebeüberzug definiert, der ein erstes und zweites gegenüberliegendes Ende und einen sich dazwischen erstreckenden mittleren Abschnitt aufweist, wobei mindestens ein Ende eine Öffnung definiert, die dafür konfiguriert ist, ein Knochenimplantat aufzunehmen.

7. Implantatvorrichtung nach Anspruch 6, wobei das erste Ende eine Öffnung definiert, die dafür konfiguriert ist, ein Knochenimplantat aufzunehmen, und das zweite Ende ein geschlossenes Ende definiert.

8. Implantatvorrichtung nach Anspruch 1, wobei der Flachkörper den Netzgewebeüberzug definiert, der eine obere und untere Fläche und eine Außenkante aufweist, wobei der Flachkörper dafür konfiguriert ist, mindestens einen Abschnitt einer Außenfläche eines Knochenimplantats zu überziehen und sich daran anzupassen.

9. Implantatsystem mit:
einer Implantatvorrichtung nach Anspruch 1 und
einem Knochenimplantat mit einer Außenfläche, wobei der Hohl- oder Flachkörper mindestens einen Abschnitt der Außenfläche des Knochenimplantats überzieht und sich daran anpasst, wobei 99 % bis 20 % der überzogenen Außenfläche des Knochenimplantats durch die Öffnungen im Netzgewebe zwecks erwünschter Knochengewebeintegration freiliegen.

10. Implantatsystem nach Anspruch 9, wobei 99 % bis 40 % der überzogenen Außenfläche des Knochenimplantats durch die Öffnungen im Netzgewebe zwecks Knochengewebeintegration freiliegen.

11. Implantatsystem nach Anspruch 9, wobei die Implantatvorrichtung den Hohlkörper aufweist, der den Netzgewebeüberzug definiert, der ein erstes und zweites gegenüberliegendes Ende und einen sich dazwischen erstreckenden mittleren Abschnitt aufweist, wobei mindestens ein Ende eine Öffnung definiert, die dafür konfiguriert ist, das Knochenimplantat aufzunehmen, und wobei das Knochenimplantat in der Öffnung aufgenommen wird, so dass der Hohlkörper mindestens einen Abschnitt der Außenfläche des Knochenimplantats überzieht und sich daran anpasst.

12. Implantatsystem nach Anspruch 11, wobei das erste Ende eine Öffnung definiert, die dafür konfiguriert ist, das Knochenimplantat aufzunehmen, und das zweite Ende ein geschlossenes Ende definiert.

13. Implantatsystem nach Anspruch 9, wobei die Implantatvorrichtung den Flachkörper aufweist, der den Netzgewebeüberzug definiert, der eine obere und untere Fläche und eine Außenkante aufweist, wobei der Flachkörper mindestens einen Abschnitt der Außenfläche des Knochenimplantats überzieht und sich daran anpasst.

## Revendications

1. Dispositif d'implant comprenant :
un corps creux ou plan qui comprend (a) un tissu textile et/ou (b) un film polymère qui définit un revêtement en treillis, et un ou plusieurs agents bioactifs incorporés dans ou sur le corps creux ou plan, où (a) et (b) comporte chacun une pluralité d'ouvertures, les ouvertures ayant une dimension maximale non supérieure à 20 mm et une dimension minimale non inférieure à 0,2 mm.

2. Implant de la revendication 1, dans lequel les ouvertures ont une dimension maximale non supérieure à 5 mm et une dimension minimale non inférieure à 0,5 mm.

3. Implant de la revendication 1, dans lequel le corps creux ou plan comprend (a) le tissu tissé, tricoté ou non tissé.

4. Implant de la revendication 1, dans lequel le corps creux ou plan comprend (b) le film polymère.

5. Implant de la revendication 1, dans lequel (a) et/ou (b) est biodégradable.

6. Dispositif d'implant de la revendication 1, dans lequel le corps creux définit le revêtement en treillis qui comporte des première et deuxième extrémités opposées et une partie intermédiaire s'étendant entre celles-ci, au moins une extrémité définissant une ouverture configurée pour recevoir un implant osseux.

7. Implant de la revendication 6, dans lequel la première extrémité définit une ouverture configurée pour recevoir un implant osseux et la deuxième extrémité définit une extrémité fermée.

8. Dispositif d'implant de la revendication 1, dans lequel le corps plan définit le revêtement en treillis qui comporte des surfaces supérieure et inférieure et un bord extérieur, le corps plan configuré pour couvrir et se conformer à au moins une partie d'une surface extérieure d'un implant osseux.

9. Système d'implant comprenant :
un dispositif d'implant tel que défini dans la revendication 1, et
un implant osseux comportant une surface extérieure, le corps creux ou plan couvrant et se conformant à au moins une partie de la surface extérieure de l'implant osseux, où 99% à 20% de la surface extérieure couverte de l'implant osseux est exposée à travers les ouvertures dans le revêtement en treillis pour l'intégration de tissu osseux souhaitable.

10. Système d'implant de la revendication 9, dans lequel 99% à 40% de la surface extérieure couverte de l'implant osseux est exposée à travers les ouvertures dans le revêtement en treillis pour l'intégration de tissu osseux.

11. Système d'implant de la revendication 9, dans lequel le dispositif d'implant comporte le corps creux définissant le revêtement en treillis qui comporte des première et deuxième extrémités opposées et une partie intermédiaire s'étendant entre celles-ci, au moins une extrémité définissant une ouverture configurée pour recevoir l'implant osseux, et où l'implant osseux est reçu à l'intérieur de l'ouverture de sorte que le corps creux couvre et se conforme à au moins une partie de la surface extérieure de l'implant osseux.

12. Système d'implant de la revendication 11, dans lequel la première extrémité définit une ouverture configurée pour recevoir l'implant osseux et la deuxième extrémité définit une extrémité fermée.

13. Système d'implant de la revendication 9, dans lequel le dispositif d'implant comporte le corps plan définissant le revêtement en treillis qui comporte des surfaces supérieure et inférieure et un bord extérieur, le corps plan couvrant et se conformant à au moins une partie de la surface extérieure de l'implant osseux.
